# EUROPEAN PATENT APPLICATION

(11) **EP 4 289 949 A1**
(43) Date of publication of application: **13.12.2023**
(21) Application number: 22749015.8
(22) Date of filing: 27.01.2022
(51) Int. Cl.: C12N 9/88, A61K 38/51, A61P 31/04, C12N 15/60, A61K 35/76, A61P 17/10

(54) **BACTERIOPHAGE LYSINE, CHIMERA THEREOF AND APPLICATION THEREOF**

(30) Priority: 08.02.2021 CN 202110187030
(71) Applicant: Wuhan Lysigen Bio-Tech Company Limited, Wuhan, Hubei 430075 (CN)
(72) Inventor: ASSAF, Raz, Wuhan, Hubei 430075 (CN); MYA, Thandar, Wuhan, Hubei 430075 (CN)
(74) Representative: Michalski Hüttermann & Partner Patentanwälte mbB
(86) International application number: PCT/CN2022/074221
(87) International publication number: WO 2022/166736

(57) **Abstract**

The invention relates to the field of medical biology, and specifically provides a phage lysin or a chimera thereof used in the preparation of medicines, cosmetics or drugs for preventing, treating or improving acne or infection caused by Propionibacterium acnes or diseases related to Propionibacterium acnes. For application in medical equipment, the phage lytic enzymes include phage lytic enzymes derived from Nocardioidaceae and Propionibacteriaceae bacteria. The phage lytic enzyme or its chimera provided by the present invention can specifically and effectively kill Propionibacterium acnes while keeping other commensal bacteria intact, and can provide a safe and effective acne solution suitable for long-term use without a high risk of dysbiosis and acquired resistance.

## Description

This application claims the following priority: CN202110187030.8, filing date 2021.02.08.

### Technical field

The invention relates to the field of medical biology, and specifically provides a bacteriophage lysin with anti-Cutibacterium acnes activity, a chimera and application thereof.

### Background technique

Acne is an inflammatory disease of the skin, especially on the face, neck, shoulders, chest, back and upper arms. The pilosebaceous unit is inflamed due to the accumulation of dead skin cells due to clogging with excess oil secretion and proliferation of the pathogenic Propionibacterium acnes (C. acnes, the same below). According to the National Institutes of Health, 80 percent of people ages 11 to 30 have acne to some extent. Acne usually begins in adolescence and may last into the forties and fifties. Different factors can trigger acne breakouts such as stress, changes in hormone levels, allergens, etc. The 2010 Global Burden of Disease study estimated that acne affects 9.4% of the global population and ranks it as the eighth most prevalent disease in the world. The American Academy of Dermatology estimated that in 2013 the cost of treating acne patients and lost productivity was more than $1.2 billion. While rarely life-threaten in itself, it can cause long-lasting scarring on the skin and considerable emotional distress for patients.

Current options for treating mild acne are the use of over-the-counter topical medications containing chemicals such as benzoyl peroxide, resorcinol, salicylic acid, and sulfur, as well as prescription topical medications such as antibiotics, benzoyl peroxide, nonyl Diacids, dapsone, corticosteroids, and vitamin A derivatives called retinoids. Moderate to severe disease can be treated with prescription topical or oral medications. The goal of the intervention is to remove dead skin cells and excess oil to unclog the pilosebaceous unit and reduce the bacterial load. Since acne is a chronic disease, it requires prolonged treatment and sometimes prevention of recurrence. However, none of the existing treatment options are suitable for long-term treatment. When these medications are used for an extended period of time, even the mildest chemicals can cause allergic reactions and irritation of the skin, leading to dryness, flaking, cracking, and more. Antibiotic treatment options are increasingly limited as resistant P. acnes become more prevalent in the world. In addition, chronic antibiotic use has been shown to disturb the homeostasis of the microbiota by exerting selective pressure on resistant bacterial species. This in turn leads to myriad complications such as dysbiosis and immune abnormalities. It has been shown in the literature that the skin Staphylococcus flora of acne patients changes from mostly antibiotic-sensitive to mostly resistant during antibiotic treatment. Not only the patient himself, but also the close contacts of the patient are also affected by multidrug resistant bacteria. Notably, acne patients who received long-term antibiotic treatment were two times more likely to develop a respiratory infection. Therefore, there is an urgent need for a safer, more effective, and more suitable for long-term use of treatment options. One such approach is pathogen-specific antimicrobials, which specifically kill P. acnes without affecting other commensal bacteria, regardless of antibiotic resistance.

In addition, P. acnes has been associated with a range of invasive infections, including postoperative shoulder infections and intervertebral disc infections. Among such infections, P. acnes exhibits a slowly progressive biofilm-like infection. Bacteria found in biofilms are inherently resistant to antibiotics. A P. acnes anti-microbial with strong anti-biofilm properties is ideal for the treatment of such infections.

An alternative for pathogen-specific antimicrobials is the bacteriophage endolysin. Bacteriophages use these enzymes to destabilize the cell wall for lysis, releasing phage progeny from the interior of the cell. They are classified as peptidoglycan hydrolases that cleave various bonds in bacterial peptidoglycan. With few exceptions, each lytic enzyme targets bacteria belonging to a single genus or species. Recombinant lysin against Gram-positive bacteria have been shown to be effective bactericides capable of causing hypotonic lysis (eg PlyC against several Streptococci, PlyG against Bacillus anthracis, etc.). Given that lysin have been under selective evolutionary pressure over billions of years to continue to successfully infect cells, the peptidoglycan bonds of interest are highly conserved and unlikely to be easily altered by bacteria. This property has little chance of developing resistance to the lyase in the target bacteria, making it suitable for long-term therapeutic use. Bacterial resistance to the respective phage lytic enzymes has not been detected so far. Generally, lysin against Gram-positive bacteria are composed of one or two N-terminal catalytic domains (CD) and a C-terminal cell wall-binding domain (BD), with linkers of different lengths between the domains). Lysin are modular proteins, and chimeric lysins can be generated by pairing catalytic and binding domains from different lysins. Linkers are also variable in length and sequence. However, the major bottleneck in the development of lysin as antibacterial agents is the inability to soluble express highly active lysin. In fact, no lysin with high level soluble expression and effective killing of P. acnes has been reported so far.

Patent CN102482655A discloses an antimicrobial agent, which is composed of an endolysin with the activity of degrading the cell wall of Gram-positive bacteria and an amphiphilic peptide segment fused to the endolysin at the N-terminal or C-terminal or both ends, can be used for the treatment or prevention of Gram-positive bacterial infections, as a diagnostic means or as a cosmetic substance. However, although the patent describes that PA6 has anti-P. acnes DSMZ 1897 strain and DSMZ 16379 strain activity, it does not provide the initial cfu/mL; in addition, PA6 cannot solve the problem of increasing the soluble expression level.

In summary, there is clearly a great need for new therapeutic modalities against P. acnes, and so far no phage lysin with high P. acnes killing activity suitable for industrial scale production has been reported. The ability of phage lytic enzymes to specifically and efficiently kill P. acnes while leaving other commensal bacteria intact may provide a safe and effective acne solution for long-term use without high dysbiosis and gain risk of drug resistance.

### Contents of the invention

Aiming at the above technical status, the present invention provides phage lysin with anti-Propionibacterium acnes activity, its chimera and application. The details of the invention are as follows:
The invention provides the use of a phage lysin or its chimera in the preparation of medicines, cosmetics, or medical devices for preventing, treating or improving acne or infection caused by Propionibacterium acnes or diseases related to Propionibacterium acnes, the bacteriophage lysin includes bacteriophage lysin derived from Nocardioidaceae and Propionibacteriaceae bacteria.

In the use of the present invention, as one of the embodiments, the Nocardioidaceae bacteria include Micropruina, Propionicimonas, and Propionicimonas ,Propionicicella, Friedmanniella; the bacteria of the family Propionibacteriaceae include the Propioniferax, Mariniluteicoccus, Granulicoccus, Naumannella, Propioniciclava, Auraticoccus, Microlunatus, Aestuariimicrobium, Luteococcus, Tessaracoccus, Brooklawnia, Propionimicrobium, Propionibacterium, Cutibacterium, Acidipropionibacterium, or Pseudopropionibacterium; preferably Propionibacterium, Cutibacterium, Acidipropionibacterium, or Pseudopropionibacterium bacteria.

In the application of the present invention, as one of the embodiments, the bacteriophage lysin includes lysins derived from Cutibacterium acnes, Propionibacterium humerusii, Cutibacterium avidum, Cutibacterium granulosum, Acidipropionibacterium thoenii, Acidipropionibacterium jensenii, Acidipropionibacterium acidipropionici, Aestuariimicrobium kwangyangense, Granulicoccus phenolivorans, Microlunatus phosphovorus, Pseudopropionibacterium propionicum, Tessaracoccus sp., Propionicicella superfundia, Propionibacterium freudenreichii, Propionibacterium freudenreichii subsp. Freudenreichii, Propionibacterium freudenreichii subsp. Shermanii, Propionibacterium acidifaciens, Propionibacterium lymphophilum, Propionibacteriaceae bacterium, Propionibacterium sp. oral taxon 192, Propioniferax innocua, Naumannella halotolerans, Propioniciclava tarda, Micropruina glycogenica, Propionicimonas paludicola, Auraticoccus monumenti, Luteococcus japonicus, Tessaracoccus oleiagri, Tessaracoccus bendigoensis, Tessaracoccus lapidicaptus, Acidipropionibacterium microaerophilum, Acidipropionibacterium olivae, Acidipropionibacterium damnosum.

In the application of the present invention, as one of the embodiments, the bacteriophage lysin comprises lysin derived from Acidipropionibacterium jensenii, Acidipropionibacterium thoenii, Acidipropionibacterium acidipropionici, Acidipropionibacterium microaerophilum, Acidipropionibacterium olivae, Acidipropionibacterium damnosum, Cutibacterium acnes, Cutibacterium avidum, Cutibacterium granulosum and Pseudopropionibacterium propionicum.
In the use of the present invention, as one of the embodiments, the bacteriophage lysin has the amino acid sequence shown in any one of SEQ ID NO:1~SEQ ID NO:28;
In the use of the present invention, as one of the embodiments, the bacteriophage lysin has the nucleotide sequence shown in any one of SEQ ID NO:29~ SEQ ID NO:56;
In the use of the present invention, as one of the embodiments, the bacteriophage lysin preferably has PACL10 having the amino acid sequence shown in SEQ ID NO:10;
In the use of the present invention, as one of the embodiments, the bacteriophage lysin is preferably PACL10 having the nucleotide sequence shown in SEQ ID NO:38.

In the use of the present invention, as one of the embodiments, the chimera comprises a catalytic domain derived from bacteriophage lysin, or a combination of a catalytic domain and a binding domain derived from bacteriophage lysin.

In the use of the present invention, as one of the embodiments, the catalytic domain has a full C-terminal linker, a half C-terminal linker, no C-terminal linker, or any part of the linker; the binding domain has full N-terminal linker, half N-terminal linker, no N-terminal linker, or any part of the linkers.

In the application of the present invention, as one of the embodiments, a synthetic linker may be used, which also includes a synthetic linker. Non-limiting examples of possible linkers derived from phage lysin are given in Table 3 below. See Table 4 below for non-limiting examples of synthetic linkers.

In the use of the present invention, as one of the embodiments, the chimera is formed by pairing catalytic domains and binding domains derived from different bacteriophage lysins.

In the application of the present invention, as one of the embodiments, the chimera includes one or two catalytic domains without a binding domain;
As one of the embodiments, the chimera includes one or more catalytic domains and one or more binding domains;
As one of the embodiments, the chimera can be one or more catalytic domains connected to a single binding domain;
As one of the embodiments, the chimera can be a single catalytic domain connected to a single binding domain;
As one of the embodiments, the chimera can be a single catalytic domain in the N-terminal of the lysin linked to a single binding domain in the C-terminal of the lysin.

Furthermore, one or more catalytic domains may be used in the absence of a binding domain. Further, for specialized applications such as detection, one or more binding domains can be used in the absence of the catalytic domain and fused to a different molecule to facilitate detection. Such molecules include but are not limited to biotin, flag tag, myc tag, avidin, streptavidin, ovalbumin, firefly luciferase, biotin, fluorescent molecules such as FITC, TRITC, Alexafluor, etc.

In the use of the present invention, as one of the embodiments, the chimera further comprises a linker between the catalytic domain and the binding domain, and the linker includes:
1) the linker region or any part thereof derived from the parent lysin molecule, the catalytic domain is derived from the parent lysin molecule;
   In the application of the present invention, as one of the embodiments, the linker region derived from the parent lyase molecule has the amino acid sequence shown in any one of SEQ ID NO:343~SEQ ID NO:367.
2) the linker region or any part thereof derived from the parent lysin molecule, the binding domain is derived from the parent lysin molecule;
   In the use of the present invention, as one of the embodiments, the linker region derived from the parent lysin molecule has any nucleotide sequence shown in SEQ ID NO: 368~SEQ ID NO: 392; or
3) The amino acid sequence shown in any one of SEQ ID NO:393~SEQ ID NO:406, or the synthetic linker domains be (GGGS)n, (GGGGS)n, (GGGGGS)n, (Gly)3-8, (EAAAK)n, (Ala-Pro)n, A(EAAAK)nALEA(EAAAK) nA amino acid sequences (wherein 1≤n≤15, n is an integer), as an exemplary illustration, n can be 1, 2, 3, 4 , 5, 6, 7, 8, 9, 10, 11, 12, 13, 14 or 15.

In the use of the present invention, as one of the embodiments, the chimera includes that the catalytic domain has the amino acid sequence shown in SEQ ID NO:57, SEQ ID NO:58, SEQ ID NO:59, SEQ ID NO:61, SEQ ID NO:62, SEQ ID NO:63, SEQ ID NO:64, SEQ ID NO:65, SEQ ID NO:66, SEQ ID NO:67, SEQ ID NO:69, SEQ ID NO:70, SEQ ID NO:71, SEQ ID NO:73, SEQID NO:74, SEQID NO:77, SEQ ID NO:80, SEQID NO:81, SEQID NO:85, SEQID NO:86, SEQID NO:87, SEQID NO:91, SEQID NO:93, SEQID NO:95, SEQ ID NO:97, SEQ ID NO:98, SEQ ID NO:99, SEQ ID NO:100, SEQ ID NO:102, SEQ ID NO:104, SEQ ID NO:105, SEQ ID NO:106. SEQ ID NO:107, SEQ ID NO:108. SEQ ID NO:110, SEQ ID NO:111, SEQ ID NO:113. SEQ ID NO:114, SEQ ID NO:117, SEQ ID NO:118, SEQ ID NO:121, SEQ ID NO:122, SEQ ID NO:125, SEQ ID NO:127, SEQ ID NO:128, SEQ ID NO:131, SEQ ID NO:132, SEQ ID NO:133, SEQ ID NO:135 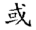 SEQ ID NO:136.

The binding domain has the amino acid sequence shown as SEQ ID NO:60, SEQ ID NO:62, SEQ ID NO:68, SEQ ID NO:72, SEQ ID NO:75, SEQ ID NO:76, SEQ ID NO:78, SEQ ID NO:79, SEQ ID NO:82, SEQ ID NO:83, SEQ ID NO:84, SEQ ID NO:88, SEQ ID NO:89, SEQ ID NO:90, SEQ ID NO:92, SEQ ID NO:94, SEQ ID NO:96, SEQ ID NO:101, SEQ ID NO:103, SEQ ID NO:109. SEQ ID NO:112, SEQ ID NO:115. SEQ ID NO:116. SEQ ID NO:119, SEQ ID NO:120. SEQ ID NO:123, SEQ ID NO:124. SEQ ID NO:126, SEQ ID NO:129, SEQ ID NO:130, SEQ ID NO:134, SEQ ID NO: 137 , SEQ ID NO:138, or SEQ ID NO:139.

In the application of the present invention, as one of the embodiments, the catalytic domain has the nucleotide sequence shown in SEQ ID NO: 140, SEQ ID NO: 141, SEQ ID NO: 142, SEQ ID NO: 144, SEQ ID NO: 146, SEQ ID NO: 147, SEQ ID NO: 148, SEQ ID NO: 149, SEQ ID NO: 150, SEQ ID NO: 152, SEQ ID NO: 153, SEQ ID NO: 154, SEQ ID NO: 156, SEQ ID NO: 157, SEQ ID NO: 160, SEQ ID NO: 163, SEQ ID NO: 164, SEQ ID NO: 168, SEQ ID NO: 169, SEQ ID NO: 170, SEQ ID NO: 174, SEQ ID NO: 176, SEQ ID NO: 178, SEQ ID NO: 180, SEQ ID NO: 181, SEQ ID NO: 182, SEQ ID NO: 183, SEQ ID NO: 185, SEQ ID NO: 187, SEQ ID NO: 188, SEQ ID NO NO: 189, SEQ ID NO: 190, SEQ ID NO: 191, SEQ ID NO:193, SEQ ID NO:194, SEQ ID NO:196, SEQ ID NO:197, SEQ ID NO:200, SEQ ID NO: 201, SEQ ID NO:204, SEQ ID NO:205, SEQ ID NO:208, SEQ ID NO:210, SEQ ID NO:211, SEQ ID NO:214, SEQ ID NO:215, SEQ ID NO:216, SEQ ID NO:218, SEQ ID NO:219;

The binding domain has the nucleotide sequence shown in SEQ ID NO:143, SEQ ID NO:145, SEQ ID NO:151, SEQ ID NO:155, SEQ ID NO:158, SEQ ID NO:159, SEQ ID NO:161, SEQ ID NO:162, SEQ ID NO:165, SEQ ID NO:166, SEQ ID NO:167, SEQ ID NO:171, SEQ ID NO:172, SEQ ID NO:173, SEQ ID NO:175, SEQ ID NO:177, SEQ ID NO:179, SEQ ID NO:184, SEQ ID NO:186, SEQ ID NO:192, SEQ ID NO:195, SEQ ID NO:198, SEQ ID NO:199, SEQ ID NO:202, SEQ ID NO:203, SEQ ID NO:206, SEQ ID NO:207. SEQ ID NO:209, SEQ ID NO:212, SEQ ID NO:213, SEQ ID NO:217, SEQ ID NO:220, SEQ ID NO:221, or SEQ ID NO:222.

In the use of the present invention, as one of the embodiments, the chimera has the amino acid sequence shown in any one of SEQ ID NO:223~SEQ ID NO:282; preferably the amino acid sequence shown in SEQ ID NO:258, SEQ ID NO:259, SEQ ID NO:260, SEQ ID NO:271 or SEQ ID NO:272, more preferably the amino acid sequence shown in SEQ ID NO:260 or SEQ ID NO:271;

In the use of the present invention, as one of the embodiments, the chimera has a nucleotide sequence shown in any one of SEQ ID NO:283~SEQ ID NO:342; preferably the nucleotide sequence shown in SEQ ID NO:316, SEQ ID NO 317, SEQ ID NO: 318, SEQ ID NO: 329 or SEQ ID NO: 330; More preferably the nucleotide sequence shown in SEQ ID NO: 318 or SEQ ID NO: 329.

In the use of the present invention, as one of the embodiments, the infection caused by Propionibacterium acnes includes invasive infection, postoperative infection and/or instrument-related infection.

In the use of the present invention, as one of the embodiments, the device-related infection includes joint prosthesis, shunt tube and artificial heart valve-related infection.

In the use of the present invention, as one of the embodiments, the infection includes bone and/or joint infection, especially postoperative shoulder infection, as well as oral cavity, eye, intervertebral disc and brain infection.

In the use of the present invention, as one of the embodiments, the diseases related to Propionibacterium acnes include prostatitis leading to cancer, SAPHO (synovitis, acne, impetigo, hypertrophy, osteitis) syndrome, knot arthritis, or sciatica.

In the use of the present invention, as one of the embodiments, the medical device includes any device for releasing the lysin or its chimera to the affected area, preferably a clamp, patch or spray applied to the skin surface, devices that use microneedles to enhance the skin penetration of lysin or their chimeras, fine needles used by cosmetic professionals to apply lysin or their chimeras specifically to acne-affected hair follicles, or other similar devices.

In the use of the present invention, as one of the embodiments, the medical device includes that fixes the lysin or its chimera in a position prone to infection of P. acnes, preferably for prosthetic implants in shoulder surgery which infected by Propionibacterium particularly.

The present invention also provides the phage lysin chimera described in the application. As one of the embodiments, the chimera has the amino acid sequence shown in any one of SEQ ID NO:223~SEQ ID NO:282; preferably the amino acid sequence shown in SEQ ID NO:258, SEQ ID NO:259, SEQ ID NO:260, SEQ ID NO:271 or SEQ ID NO:272; more preferably the amino acid sequence shown in SEQ ID NO:260 or SEQ ID NO:271;

In the application of the present invention, as one of the embodiments, the chimera has a nucleotide sequence shown in any one of SEQ ID NO:283~SEQ ID NO:342; preferably the nucleotide sequence shown in SEQ ID NO:316, SEQ ID NO: 317, SEQ ID NO: 318, SEQ ID NO: 329 or SEQ ID NO: 330; More preferably the nucleotide sequence shown in SEQ ID NO: 318 or SEQ ID NO: 329.

The present invention also provides a method for preparing the aforementioned chimera, comprising:
(1) Synthetic domain sequences and primers for amplifying domain sequences;
(2) Using Taq DNA polymerase PCR to amplify the domain sequence;
(3) The PCR product was gel-purified and ligated with the expression plasmid pET28;
(4) Transfer the recombinant plasmid to Escherichia coli BL21 (DE3);
(5) Cultivate Escherichia coli BL21 (DE3) containing the recombinant plasmid, induce expression, collect the cells by centrifugation, lyse, and purify to obtain the chimera.

In the preparing method of the present invention, as one of the embodiments, the method further includes:
Escherichia coli BL21 (DE3) containing recombinant chimeric lysin expression plasmid was cultured in self-inducing medium at 37°C and 300 rpm until the OD₆₀₀ reached 0.6-0.8, and then at 18°C and 300 rpm for continuous incubate for 16-18 hours. Cells were collected by centrifugation, resuspended in 50mM sodium phosphate pH 7.4, and lysed by homogenization under high pressure. The lysin was centrifuged again to collect the soluble crude lysate. The soluble fraction was mixed with an equal volume of 5 M NaCl, and the mixture was loaded onto a hydrophobic column. After sample loading, the column was washed with 5 column volumes of 20 mM sodium phosphate (pH 7.4), 2.5 M NaCl. The recombinant chimeric lyase was then eluted with 10 mM sodium phosphate (pH 7.4). Alternatively, expression and purification can be optimized by those skilled in the art, and the lysin can be expressed in LB using different concentrations of IPTG at different temperatures, aeration and induction times. Cells can be resuspended in different buffers to increase solubility and lysed by mechanical or chemical means. Different protein chromatography methods can be used to purify the lysin. These variables can be optimized for better lysin protein yield.

The present invention also provides preparations containing the chimera, which further comprises antibiotics, other lysin, or inactive excipients.

The present invention also provides the amino acid sequence encoding the chimera, which is 80% or more, 85% or more, 90% or more, 95% or more or 99% or more of the amino acid sequence similarity to the above sequence, or an alternative amino acid sequence having similar functional group. As an exemplary illustration, the present invention also provides amino acid sequences with 80%, 81%, 82%, 83%, 84%, 85%, 86%, 87%, 88%, 89%, 90%, 91 %, 92%, 93%, 94%, 95%, 96%, 97%, 98%, 99% similarity to the aforesaid sequences.

As one of the embodiments, the replacement amino acid sequence is a conservative substitution using amino acids in the same group of amino acids, and the amino acid group includes:
Aliphatic: glycine, alanine, valine, leucine, isoleucine;
Hydroxyl or sulfur/selenium: serine, cysteine, threonine, methionine;
Cyclic: proline;
Aromatic: phenylalanine, tyrosine, tryptophan;
Basic: histidine, lysine, arginine;
Acidic and their amides: aspartic acid, glutamic acid, asparagine, glutamine.

The present invention also provides the nucleotide sequence encoding the chimera, or its synonymous codon sequence.

The present invention also provides the use of the chimera as a reagent for bacterial lysis for DNA extraction and typing using a PCR-based kit.

The present invention also provides the use of the chimera as a disinfectant or sterilizing agent on an abiotic surface to prevent infection by removing P. acnes in planktonic form or in biofilms, preferably, surgical equipment or prosthetic implant devices are sterilized during the surgical procedure.

The present invention also provides an application of the chimera, a single binding domain in the chimera, or a series combination of similar or different binding domains in the chimera in the preparation of a diagnostic tool for P. acnes, wherein the lysin, chimera or binding domain is used in combination with a detection marker.

As one of the embodiments, the lysin, the chimera or the binding domain and the signal molecule form a fusion through gene fusion or chemical coupling.

As one of the embodiments, the fusion is used to directly detect Propionibacterium acnes on a microscope slide by fluorescence or other means, to label Propionibacterium acnes by immunohistochemistry, and to be used as a detection reagent in an ELISA assay, Use as a detection reagent on Western blot, for attachment to magnetic beads in MACS or other pull-down assays, or as a detection reagent in assays in which antibodies are used as detection reagents. As one of the embodiments, the signal molecules include proteins or chemical fluorescent dyes, protein labels, enzymes, avidin, streptavidin, ovalbumin, biotin, labels sensitive to click chemical labels, inclusion Peptides, or other molecules that can cause the recruitment of secondary proteins or molecules that produce signals,
The fluorescent dyes include GFP, RFP, mCherry, FITC, TRITC, Alexafluor 488, Cy3 or Cy5;
The protein tag includes Flag-tag, myc-tag, halo-tag, his-tag, or any other tag that can be combined with antibodies or other high-affinity molecules to generate signals;
The enzyme includes firefly luciferase, β-lactamase, alkaline phosphatase, horseradish peroxidase, or any other enzyme that causes reactions such as light, color change, substrate deposition or other reactions that can be detected in the assay.

The present invention also provides the use of the catalytic domain in the chimera in the preparation of a drug for treating P. acnes infection, where the catalytic domain is combined with a targeting module.

The present invention provides compositions and methods for preventing and treating P. acnes infection and skin colonization and acne lesions associated with such infection or colonization. In a broad aspect, the present invention provides the use and application of a lysin having broad killing activity against skin-related organisms involved in the development or exacerbation of acne lesions and secondary infection of acne lesions, including but not limited to Propionibacterium, Pseudopropionibacterium, Cutibacterium acnes. In particular, the present invention describes methods for the decolonization, dispersion and removal of established bacterial flora in the skin, with particular emphasis on Cutibacterium acnes, (formerly known as Propionibacterium acnes). In addition, the present invention provides methods for producing chimeric lysin comprising catalytic domains and binding domains from different lysin resulting in various advantageous properties including, but not limited to, improved activity, host range, expression, solubility, stability or more suitable for commercialization.

According to the present invention, in the method and application of the present invention, the phage lysin used is derived from Propionibacterium, Cutibacterium, Acidipropionibacterium, Pseudopropionibacterium. Table 2 provides lysin and polypeptides used in the present invention.

In one aspect of the present invention, the lysin shown in SEQ ID NO:1~SEQ ID NO:28 is encoded by the nucleotide sequence shown in SEQ ID NO:29~SEQ ID NO:56.

The present invention also provides a method for generating chimeras between the binding and catalytic domains of different phage lysin. SEQ ID NO:57~SEQ ID NO:139 shows a non-limiting sequence listing providing examples of polypeptide sequences encoding the catalytic domain and the binding domain derived from phage lysin of the present invention.

In one aspect of the invention, the catalytic domain and binding domain shown in SEQ ID NO:57~SEQ ID NO:139 are encoded by the nucleotide sequence shown in SEQ ID NO:140~SEQ ID NO:222.

The present invention also provides non-limiting examples of chimeric lysin produced by pairing the catalytic and binding domains of different lysin as described herein. SEQ ID NO:223-SEQ ID NO:282 provides a non-limiting sequence listing of chimeric lysin active against P. acnes produced by the methods of the present invention.

In one aspect of the present invention, the nucleotide sequence described in SEQ ID NO:283~SEQ ID NO:342 is used to generate the chimeric lysin described in the present invention.

In one aspect of the present invention, the polypeptide sequence is encoded by the nucleotide sequence inserted into the expression vector pET28.

In one aspect of the present invention, the expression vector encoding the polypeptide of the present invention is expressed in Escherichia coli (E. coli) BL21 (DE3) strain.

In addition, the present invention provides a method for screening and evaluating the activity of phage lysin against Propionibacterium acnes.

Furthermore, the present invention demonstrates the activity of defined phage lysin against P. acnes.

In one aspect of the invention, the lysin for treating Propionibacterium is PACL10. The invention provides a method for expressing and purifying PACL10. In addition, the present invention proves that PACL10 has higher antibacterial activity against Propionibacterium acnes.

The activity of PACL10 against a series of P. acnes strains of different clades was evaluated by minimum inhibitory concentration (MIC) test and time killing assay, and the results consistently showed that PACL10 has high effectiveness.

The phage lysin and its derivatives produced in the present invention are suitable for industrial scale production, can specifically and effectively kill Propionibacterium acnes, while keeping other commensal bacteria intact, and can provide a safe and effective acne solution, suitable for long-term use without high risk of dysbiosis and acquired resistance. The phage lysin and derivatives thereof produced in the present invention have the following advantages:
(1) It can kill Propionibacterium acnes (C. acnes) without damaging the natural flora.
(2) Rapid activity (within minutes) compared to antibiotics that take a long time to act.
(3) No drug resistance. Bacteria quickly develop resistance to antibiotics, so lysin are a solution. C. acnes is a strongly secreting biofilm organism. Even for organisms that are sensitive to antibiotics, when they are found in the form of biofilms, they can become resistant to antibiotics. Lysin is highly effective against biofilms.
(4) The invention is more efficient compared to other lyases - low MIC and high activity in other assays.
(5) It can be expressed in a soluble manner, and its yield is suitable for large-scale and industrial production.
( 6 ) Active against all clades of C. acnes..

### Definition

Due to the reclassification of Propionibacterium by Scholz et al., a non-limiting example of the comparison of old and new names is shown in Table 1 below:

**Table 1**

| Chinese Name | New Name | Old Name |
|---|---|---|
| Cuochuangbingsuanganjun | *Cutibacterium acnes* | *Propionibacterium acnes* |
| Tanlanbingsuanganjun | *Cutibacterium avidum* | *Propionibacterium avidum* |
| Kelibingsuanganjun | *Cutibacterium granulosum* | *Propionibacterium granulosum* |
| Hongbingsuanganjun | *Cutibacterium humerusii* | *Propionibacterium humerusii* |
| Teshibingsuanganjun | *Acidipropionibacterium thoenii* | *Propionibacterium thoenii* |
| Zhanshibingsuanganjun | *Acidipropionibacterium jensenii* | *Propionibacterium jensenii* |
| Chanbingsuanganjun | *Acidipropionibacterium acidipropionici* | *Propionibacterium acidipropionici* |
| Weishiqibingsuanganjun | *Acidipropionibacterium microaerophilum* | *Propionibacterium microaerophilum* |
| Dannuoshibingsuanganjun | *Acidipropionibacterium damnosum* | *Propionibacterium damnosum* |
| Ganlantibingsuanganjun | *Acidipropionibacterium olivae* | *Propionibacterium olivae* |
| bingsuanbingsuan ganjun | *Pseudopropionibacterium propionicum* | *Propionibacterium propionicum* |

Persons in the field should understand that the reclassification, new and old names, and Chinese names of bacteria should not affect the identification of the bacteria. No matter whether the new name or the old name is adopted in the present invention, it represents the same bacterial species.

Non-limiting examples of sequences related to the present invention are as follows in Table 2:

**Table 2**

| Name | Amino acid sequence | Name | Nucleotide sequence |
|---|---|---|---|
| PACL01 | SEQ ID NO:1 | PACL01 | SEQ ID NO:29 |
| PACL02 | SEQ ID NOT | PACL02 | SEQ ID NO:30 |
| PACL03 | SEQ ID NOT | PACL03 | SEQ ID NO:31 |
| PACL04 | SEQ ID NOT | PACL04 | SEQ ID NO:32 |
| PACL05 | SEQ ID NO:5 | PACL05 | SEQ ID NO:33 |
| PACL06 | SEQ ID NOT | PACL06 | SEQ ID NO:34 |
| PACL07 | SEQ ID NOT | PACL07 | SEQ ID NO:35 |
| PACL08 | SEQ ID NOT | PACL08 | SEQ ID NO:36 |
| PACL09 | SEQ ID NO:9 | PACL09 | SEQ ID NO:37 |
| PACL10 | SEQ ID NO:10 | PACL10 | SEQ ID NO:38 |
| PACL 11 | SEQ ID NO:11 | PACL 11 | SEQ ID NO:39 |
| PACL12 | SEQ ID NO:12 | PACL12 | SEQ ID NO:40 |
| PACL13 | SEQ ID NO:13 | PACL13 | SEQ ID NO:41 |
| PACL14 | SEQ ID NO:14 | PACL14 | SEQ ID NO:42 |
| PACL15 | SEQ ID NO:15 | PACL15 | SEQ ID NO:43 |
| PACL16 | SEQ ID NO:16 | PACL16 | SEQ ID NO:44 |
| PACL17 | SEQ ID NO:17 | PACL17 | SEQ ID NO:45 |
| PACL18 | SEQ ID NO:18 | PACL18 | SEQ ID NO:46 |
| PACL19 | SEQ ID NO:19 | PACL19 | SEQ ID NO:47 |
| PACL20 | SEQ ID NO:20 | PACL20 | SEQ ID NO:48 |
| PACL21 | SEQ ID NO:21 | PACL21 | SEQ ID NO:49 |
| PACL22 | SEQ ID NO:22 | PACL22 | SEQ ID NO:50 |
| PACL23 | SEQ ID NO:23 | PACL23 | SEQ ID NO:51 |
| PACL24 | SEQ ID NO:24 | PACL24 | SEQ ID NO:52 |
| PACL25 | SEQ ID NO:25 | PACL25 | SEQ ID NO:53 |
| PACL26 | SEQ ID NO:26 | PACL26 | SEQ ID NO:54 |
| PACL27 | SEQ ID NO:27 | PACL27 | SEQ ID NO:55 |
| PACL28 | SEQ ID NO:28 | PACL28 | SEQ ID NO:56 |
| | | | |
| PACL01 CD1 | SEQ ID NO:57 | PACL01 CD1 | SEQ ID NO:140 |
| PACL01 CD2 | SEQ ID NO:58 | PACL01 CD2 | SEQ ID NO:141 |
| PACL01 CD1_2 | SEQ ID NO:59 | PACL01 CD1_2 | SEQ ID NO:142 |
| PACL01 BD | SEQ ID NO:60 | PACL01 BD | SEQ ID NO:143 |
| | | | |
| PACL02 CD | SEQ ID NO:61 | PACL02 CD | SEQ ID NO:144 |
| PACL02 BD | SEQ ID NO:62 | PACL02 BD | SEQ ID NO:145 |
| | | | |
| PACL03 CD | SEQ ID NO:63 | PACL03 CD | SEQ ID NO:146 |
| | | | |
| PACL04 CD | SEQ ID NO:64 | PACL04 CD | SEQ ID NO:147 |
| | | | |
| PACL05 CD1 | SEQ ID NO:65 | PACL05 CD1 | SEQ ID NO:148 |
| PACL05 CD2 | SEQ ID NO:66 | PACL05 CD2 | SEQ ID NO:149 |
| PACL05 CD1_2 | SEQ ID NO:67 | PACL05 CD1_2 | SEQ ID NO:150 |
| PACL05 BD | SEQ ID NO:68 | PACL05 BD | SEQ ID NO:151 |
| | | | |
| PACL06 CD1 | SEQ ID NO:69 | PACL06 CD1 | SEQ ID NO:152 |
| PACL06 CD2 | SEQ ID NO:70 | PACL06 CD2 | SEQ ID NO:153 |
| PACL06 CD1 _ 2 | SEQ ID NO:71 | PACL06 CD1_2 | SEQ ID NO:154 |
| PACL06 BD | SEQ ID NO:72 | PACL06 BD | SEQ ID NO:155 |
| | | | |
| PACL07 CDf | SEQ ID NO:73 | PACL07 CDf | SEQ ID NO:156 |
| PACL07CD0 | SEQ ID NO:74 | PACL07CD0 | SEQ ID NO:157 |
| PACL07 BDf | SEQ ID NO:75 | PACL07 BDf | SEQ ID NO:158 |
| PACL07BD0 | SEQ ID NO:76 | PACL07BD0 | SEQ ID NO:159 |
| | | | |
| PACL08 CD | SEQ ID NO:77 | PACL08 CD | SEQ ID NO:160 |
| PACL08 BDf | SEQ ID NO:78 | PACL08 BDf | SEQ ID NO:161 |
| PACL08BD0.5 | SEQ ID NO:79 | PACL08BD0.5 | SEQ ID NO:162 |
| | | | |
| PACL09 CDf | SEQ ID NO:80 | PACL09 CDf | SEQ ID NO:163 |
| PACL09CD0 | SEQ ID NO:81 | PACL09CD0 | SEQ ID NO:164 |
| PACL09 BDf | SEQ ID NO:82 | PACL09 BDf | SEQ ID NO:165 |
| PACL09BD0 | SEQ ID NO:83 | PACL09BD0 | SEQ ID NO:166 |
| PACL09BD0.5 | SEQ ID NO:84 | PACL09BD0.5 | SEQ ID NO:167 |
| | | | |
| PACL10 CDf | SEQ ID NO:85 | PACL10 CDf | SEQ ID NO:168 |
| PACL10CD0 | SEQ ID NO:86 | PACL10CD0 | SEQ ID NO:169 |
| PACL10CD0.5 | SEQ ID NO:87 | PACL10CD0.5 | SEQ ID NO:170 |
| PACL10 BDf | SEQ ID NO:88 | PACL10 BDf | SEQ ID NO:171 |
| PACL10BD0 | SEQ ID NO:89 | PACL10BD0 | SEQ ID NO:172 |
| PACL10BD0.5 | SEQ ID NO:90 | PACL10BD0.5 | SEQ ID NO:173 |
| | | | |
| PACL11 CD | SEQ ID NO:91 | PACL11 CD | SEQ ID NO:174 |
| PACL11 BD | SEQ ID NO:92 | PACL11 BD | SEQ ID NO:175 |
| | | | |
| PACL12 CD | SEQ ID NO:93 | PACL12 CD | SEQ ID NO:176 |
| PACL12 BD | SEQ ID NO:94 | PACL12 BD | SEQ ID NO:177 |
| | | | |
| PACL13 CD | SEQ ID NO:95 | PACL13 CD | SEQ ID NO:178 |
| PACL13 BD | SEQ ID NO:96 | PACL13 BD | SEQ ID NO:179 |
| | | | |
| PACL14 CD 1 | SEQ ID NO:97 | PACL14 CD1 | SEQ ID NO:180 |
| PACL14 CD2 | SEQ ID NO:98 | PACL14 CD2 | SEQ ID NO:181 |
| PACL14 CD | SEQ ID NO:99 | PACL14 CD | SEQ ID NO:182 |
| | | | |
| PACL15 CD | SEQ ID NO:100 | PACL15 CD | SEQ ID NO:183 |
| PACL15 BD | SEQ ID NO:101 | PACL15 BD | SEQ ID NO:184 |
| | | | |
| PACL16 CD | SEQ ID NO:102 | PACL16 CD | SEQ ID NO:185 |
| PACL16 BD | SEQ ID NO:103 | PACL16 BD | SEQ ID NO:186 |
| | | | |
| PACL17 CD 1 | SEQ ID NO:104 | PACL17 CD1 | SEQ ID NO:187 |
| PACL17 CD | SEQ ID NO:105 | PACL17 CD | SEQ ID NO:188 |
| | | | |
| PACL18 CD1 | SEQ ID NO:106 | PACL18 CD1 | SEQ ID NO:189 |
| PACL18 CD | SEQ ID NO:107 | PACL18 CD | SEQ ID NO:190 |
| | | | |
| PACL19 CD | SEQ ID NO:108 | PACL19 CD | SEQ ID NO:191 |
| PACL19 BD | SEQ ID NO:109 | PACL19 BD | SEQ ID NO:192 |
| | | | |
| PACL20 CDf | SEQ ID NO:110 | PACL20 CDf | SEQ ID NO:193 |
| PACL20CD0 | SEQ ID NO:111 | PACL20CD0 | SEQ ID NO:194 |
| PACL20 BD | SEQ ID NO:112 | PACL20 BD | SEQ ID NO:195 |
| | | | |
| PACL21 CDf | SEQ ID NO:113 | PACL21 CDf | SEQ ID NO:196 |
| PACL21CD0 | SEQ ID NO:114 | PACL21CD0 | SEQ ID NO:197 |
| PACL21 BDf | SEQ ID NO:115 | PACL21 BDf | SEQ ID NO:198 |
| PACL21BD0 | SEQ ID NO:116 | PACL21BD0 | SEQ ID NO:199 |
| | | | |
| PACL22 CDf | SEQ ID NO:117 | PACL22 CDf | SEQ ID NO:200 |
| PACL22CD0 | SEQ ID NO:118 | PACL22CD0 | SEQ ID NO:201 |
| PACL22 BDf | SEQ ID NO:119 | PACL22 BDf | SEQ ID NO:202 |
| PACL22BD0 | SEQ ID NO:120 | PACL22BD0 | SEQ ID NO:203 |
| | | | |
| PACL23 CDf | SEQ ID NO:121 | PACL23 CDf | SEQ ID NO:204 |
| PACL23CD0 | SEQ ID NO:122 | PACL23CD0 | SEQ ID NO:205 |
| PACL23 BDf | SEQ ID NO:123 | PACL23 BDf | SEQ ID NO:206 |
| PACL23BD0 | SEQ ID NO:124 | PACL23BD0 | SEQ ID NO:207 |
| | | | |
| PACL24 CD | SEQ ID NO:125 | PACL24 CD | SEQ ID NO:208 |
| PACL24 BD | SEQ ID NO:126 | PACL24 BD | SEQ ID NO:209 |
| | | | |
| PACL25 CDf | SEQ ID NO:127 | PACL25 CDf | SEQ ID NO:210 |
| PACL25CD0 | SEQ ID NO:128 | PACL25CD0 | SEQ ID NO:211 |
| PACL25 BDf | SEQ ID NO:129 | PACL25 BDf | SEQ ID NO:212 |
| PACL25BD0.5 | SEQ ID NO:130 | PACL25BD0.5 | SEQ ID NO:213 |
| | | | |
| PACL26 CDf | SEQ ID NO:131 | PACL26 CDf | SEQ ID NO:214 |
| PACL26CD0 | SEQ ID NO:132 | PACL26CD0 | SEQ ID NO:215 |
| PACL26CD0.5 | SEQ ID NO:133 | PACL26CD0.5 | SEQ ID NO:216 |
| PACL26 BD | SEQ ID NO:134 | PACL26 BD | SEQ ID NO:217 |
| | | | |
| PACL27 CDf | SEQ ID NO:135 | PACL27 CDf | SEQ ID NO:218 |
| PACL27CD0 | SEQ ID NO:136 | PACL27CD0 | SEQ ID NO:219 |
| PACL27 BDf | SEQ ID NO:137 | PACL27 BDf | SEQ ID NO:220 |
| PACL27BD0 | SEQ ID NO:138 | PACL27BD0 | SEQ ID NO:221 |
| PACL27BD0.5 | SEQ ID NO:139 | PACL27BD0.5 | SEQ ID NO:222 |
| | | | |
| PACL246 | SEQ ID NO:223 | PACL246 | SEQ ID NO:283 |
| PACL247 | SEQ ID NO:224 | PACL247 | SEQ ID NO:284 |
| PACL248 | SEQ ID NO:225 | PACL248 | SEQ ID NO:285 |
| PACL249 | SEQ ID NO:226 | PACL249 | SEQ ID NO:286 |
| PACL250 | SEQ ID NO:227 | PACL250 | SEQ ID NO:287 |
| PACL251 | SEQ ID NO:228 | PACL251 | SEQ ID NO:288 |
| PACL252 | SEQ ID NO:229 | PACL252 | SEQ ID NO:289 |
| PACL253 | SEQ ID NO:230 | PACL253 | SEQ ID NO:290 |
| PACL254 | SEQ ID NO:231 | PACL254 | SEQ ID NO:291 |
| PACL255 | SEQ ID NO:232 | PACL255 | SEQ ID NO:292 |
| PACL256 | SEQ ID NO:233 | PACL256 | SEQ ID NO:341 |
| PACL262 | SEQ ID NO:234 | PACL262 | SEQ ID NO:293 |
| PACL269 | SEQ ID NO:235 | PACL269 | SEQ ID NO:294 |
| PACL287 | SEQ ID NO:236 | PACL287 | SEQ ID NO:295 |
| PACL289 | SEQ ID NO:237 | PACL289 | SEQ ID NO:296 |
| PACL296 | SEQ ID NO:238 | PACL296 | SEQ ID NO:297 |
| PACL306 | SEQ ID NO:239 | PACL306 | SEQ ID NO:342 |
| PACL341 | SEQ ID NO:240 | PACL341 | SEQ ID NO:298 |
| PACL342 | SEQ ID NO:241 | PACL342 | SEQ ID NO:299 |
| PACL343 | SEQ ID NO:242 | PACL343 | SEQ ID NO:300 |
| PACL344 | SEQ ID NO:243 | PACL344 | SEQ ID NO:301 |
| PACL345 | SEQ ID NO:244 | PACL345 | SEQ ID NO:302 |
| PACL347 | SEQ ID NO:245 | PACL347 | SEQ ID NO:303 |
| PACL348 | SEQ ID NO:246 | PACL348 | SEQ ID NO:304 |
| PACL349 | SEQ ID NO:247 | PACL349 | SEQ ID NO:305 |
| PACL350 | SEQ ID NO:248 | PACL350 | SEQ ID NO:306 |
| PACL351 | SEQ ID NO:249 | PACL351 | SEQ ID NO:307 |
| PACL352 | SEQ ID NO:250 | PACL352 | SEQ ID NO:308 |
| PACL383 | SEQ ID NO:251 | PACL383 | SEQ ID NO:309 |
| PACL384 | SEQ ID NO:252 | PACL384 | SEQ ID NO:310 |
| PACL385 | SEQ ID NO:253 | PACL385 | SEQ ID NO:311 |
| PACL386 | SEQ ID NO:254 | PACL386 | SEQ ID NO:312 |
| PACL387 | SEQ ID NO:255 | PACL387 | SEQ ID NO:313 |
| PACL388 | SEQ ID NO:256 | PACL388 | SEQ ID NO:314 |
| PACL389 | SEQ ID NO:257 | PACL389 | SEQ ID NO:315 |
| PACL390 | SEQ ID NO:258 | PACL390 | SEQ ID NO:316 |
| PACL391 | SEQ ID NO:259 | PACL391 | SEQ ID NO:317 |
| PACL392 | SEQ ID NO:260 | PACL392 | SEQ ID NO:318 |
| PACL393 | SEQ ID NO:261 | PACL393 | SEQ ID NO:319 |
| PACL394 | SEQ ID NO:262 | PACL394 | SEQ ID NO:320 |
| PACL395 | SEQ ID NO:263 | PACL395 | SEQ ID NO:321 |
| PACL396 | SEQ ID NO:264 | PACL396 | SEQ ID NO:322 |
| PACL397 | SEQ ID NO:265 | PACL397 | SEQ ID NO:323 |
| PACL398 | SEQ ID NO:266 | PACL398 | SEQ ID NO:324 |
| PACL399 | SEQ ID NO:267 | PACL399 | SEQ ID NO:325 |
| PACL400 | SEQ ID NO:268 | PACL400 | SEQ ID NO:326 |
| PACL401 | SEQ ID NO:269 | PACL401 | SEQ ID NO:327 |
| PACL402 | SEQ ID NO:270 | PACL402 | SEQ ID NO:328 |
| PACL403 | SEQ ID NO:271 | PACL403 | SEQ ID NO:329 |
| PACL404 | SEQ ID NO:272 | PACL404 | SEQ ID NO:330 |
| PACL405 | SEQ ID NO:273 | PACL405 | SEQ ID NO:331 |
| PACL406 | SEQ ID NO:274 | PACL406 | SEQ ID NO:332 |
| PACL407 | SEQ ID NO:275 | PACL407 | SEQ ID NO:333 |
| PACL408 | SEQ ID NO:276 | PACL408 | SEQ ID NO:334 |
| PACL409 | SEQ ID NO:277 | PACL409 | SEQ ID NO:335 |
| PACL410 | SEQ ID NO:278 | PACL410 | SEQ ID NO:336 |
| PACL411 | SEQ ID NO:279 | PACL411 | SEQ ID NO:337 |
| PACL412 | SEQ ID NO:280 | PACL412 | SEQ ID NO:338 |
| PACL413 | SEQ ID NO:281 | PACL413 | SEQ ID NO:339 |
| PACL414 | SEQ ID NO:282 | PACL414 | SEQ ID NO:340 |

Non-limiting examples of natural linkers described in the present invention are shown in Table 3 below:

**Table 3**

| Original Lysin | Linker Name | Amino Acid Sequence | Nucleic Acid Sequence |
|---|---|---|---|
| PACL01 | 01linker1 | SEQ ID NO:343 | SEQ ID NO:368 |
| PACL01 | 01linker2 | SEQ ID NO:344 | SEQ ID NO:369 |
| PACL02 | 02linker | SEQ ID NO:345 | SEQ ID NO:370 |
| PACL05 | 05linker1 | SEQ ID NO:346 | SEQ ID NO:371 |
| PACL05 | 05linker2 | SEQ ID NO:347 | SEQ ID NO:372 |
| PACL06 | 06linker1 | SEQ ID NO:348 | SEQ ID NO:373 |
| PACL06 | 06linker2 | SEQ ID NO:349 | SEQ ID NO:374 |
| PACL07 | 07 linker | SEQ ID NO:350 | SEQ ID NO:375 |
| PACL08 | 08linker | SEQ ID NO:351 | SEQ ID NO:376 |
| PACL09 | 09linker | SEQ ID NO:352 | SEQ ID NO:377 |
| PACL10 | 10linker | SEQ ID NO:353 | SEQ ID NO:378 |
| PACL11 | 11linker | SEQ ID NO:354 | SEQ ID NO:379 |
| PACL12 | 12linker | SEQ ID NO:355 | SEQ ID NO:380 |
| PACL13 | 13linker | SEQ ID NO:356 | SEQ ID NO:381 |
| PACL14 | 14linker | SEQ ID NO:357 | SEQ ID NO:382 |
| PACL15 | 15linker | SEQ ID NO:358 | SEQ ID NO:383 |
| PACL16 | 16linker | SEQ ID NO:359 | SEQ ID NO:384 |
| PACL19 | 19linker | SEQ ID NO:360 | SEQ ID NO:385 |
| PACL20 | 20linker | SEQ ID NO:361 | SEQ ID NO:386 |
| PACL21 | 21linker | SEQ ID NO:362 | SEQ ID NO:387 |
| PACL22 | 22linker | SEQ ID NO:363 | SEQ ID NO:388 |
| PACL23 | 23linker | SEQ ID NO:364 | SEQ ID NO:389 |
| PACL25 | 25linker | SEQ ID NO:365 | SEQ ID NO:390 |
| PACL26 | 26linker | SEQ ID NO:366 | SEQ ID NO:391 |
| PACL27 | 27linker | SEQ ID NO:367 | SEQ ID NO:392 |

Non-limiting examples of synthetic linkers described in the present invention are shown in Table 4 below:

**Table 4**

| S/N | Linker Name | Amino Acid Sequence |
|---|---|---|
| 1 | (GGGS)n, 1≤n≤8, n is an integer | - |
| 2 | (GGGGS)n, 1≤n≤8, n is an integer | - |
| 3 | (GGGGGS)n, 1≤n≤8, n is an integer | - |
| 4 | (Gly)₃₋₈ | - |
| 5 | (EAAAK)n, 1≤n≤8, n is an integer | - |
| 6 | PAPAP | SEQ ID NO:393 |
| 7 | AEAAAKEAAAKA | SEQ ID NO:394 |
| 8 | (Ala-Pro)ₙ, 1≤n≤15, n is an integer | - |
| 9 | A(EAAAK)ₙALEA(EAAAK)ₙA, 1≤n≤8, n is an integer | - |
| 10 | VSQTSKLTRAETVFPDV | SEQ ID NO:395 |
| 11 | PLG LWA | SEQ ID NO:396 |
| 12 | RVLAEA | SEQ ID NO:397 |
| 13 | EDVVCCSMSY | SEQ ID NO:398 |
| 14 | GGIEGRGS | SEQ ID NO:399 |
| 15 | TRHRQPRGWE | SEQ ID NO:400 |
| 16 | AGNRVRRSVG | SEQ ID NO:401 |
| 17 | RRRRRRRRR | SEQ ID NO:402 |
| 18 | GFLG | SEQ ID NO:403 |
| 19 | KESGSVSSEQLAQFRSLD | SEQ ID NO:404 |
| 20 | EGKSSGSGSESKST | SEQ ID NO:405 |
| 21 | GSAGSAAGSGEF | SEQ ID NO:406 |

### Description of drawings

Figure 1: Example 3 shows the effective killing effect of selected lysin on P. acnes. Lysin was expressed in E. coli BL21(DE3) on LB agar containing IPTG and released by E. coli osmotic lysis. A clearing zone or halo around the E. coli indicates that the expressed lysin inhibits growth or kills P. acnes embedded in the agar overlay.
Figure 2: Example 3 shows the clearing zone formed on the P. acnes overlay by crude E. coli lysates containing induced selected lysin.
Figure 3: Example 4 demonstrates that PACL10 can be soluble expressed and purified. All samples were run at 150 V for 50 mins in a 5-12% tris-glycine gel and stained with Coomassie brilliant blue. PACL10 was expressed in E. coli BL21 (DE3) using the pET expression system and purified by hydrophobic interaction. The eluted fractions show purified PACL10 at 29.4 kDa.
Figure 4: Example 7 shows the kinetics of PACL10 in reducing cfu/mL of P. acnes 34A strain. When the MIC was 6.4 µg/mL and the 2x MIC was 12.8 µg/mL, the cfu/mL could drop below the detection limit within 3 hours and 2 hours, respectively.
Figure 5: shows a single-step purified lysin for MIC determination. Lysin concentrations loaded on 5-12% Tris-HCl SDS gels were: 0.62 mg/mL PACL10, 1.41 mg/mL PACL390, 2.12 mg/mL PACL391, 2.10 mg/mL PACL392, 1.49 mg/mL PACL403 and 1.60 mg/mL PACL404.
Figure 6: shows PACL392 purified by mixed mode and ion exchange chromatography.
Figure 7: shows the bactericidal activity of PACL392 relative to strain 10.
Figure 8: shows PACL403 purified by mixed mode chromatography.
Figure 9: shows the bactericidal activity of PACL403 relative to strain 10 - OD reduction.
Figure 10: shows the bactericidal activity of PACL403 relative to strain 10 - CFU reduction.
Figure 11: shows the bactericidal activity of PACL403 relative to biofilm-associated strain 10.

### Specific embodiment

The following examples and/or experimental examples are only used to further illustrate the present invention, but do not limit the effective scope of the present invention in any way. Experiments were carried out using standard experimental methods to obtain the results shown in the examples.

### Example 1

By analyzing the sequences of prophages in the genomes of Propionibacteriaceae bacteria, we identified P. acnes phage lysin that could be soluble expressed and purified. The gene sequence expressing phage lysin was synthesized by Sangon and ligated with the expression plasmid pET28. Each recombinant plasmid was then transformed into E. coli BL21 (DE3) strain.

### Example 2

The chimera was constructed as an N-terminal catalytic domain (CD) and a C-terminal binding domain (BD), with a variable-length linker in between. Domains were identified by sequence analysis using the NCBI constant region database and the RaptorX web server for in silico protein structure prediction. The catalytic domain has a full C-terminal, half C-terminal or no C-terminal linker. Likewise, the binding domain has a full N-terminal, half N-terminal or no N-terminal linkage. Primers for amplifying domain sequences were synthesized by GeneCreate. Domain sequences were amplified using Taq DNA polymerase PCR at an annealing temperature of 55°C. The PCR product was gel purified and ligated with the expression plasmid pET28. The recombinant plasmid was then transferred to E. coli BL21 (DE3).

### Example 3

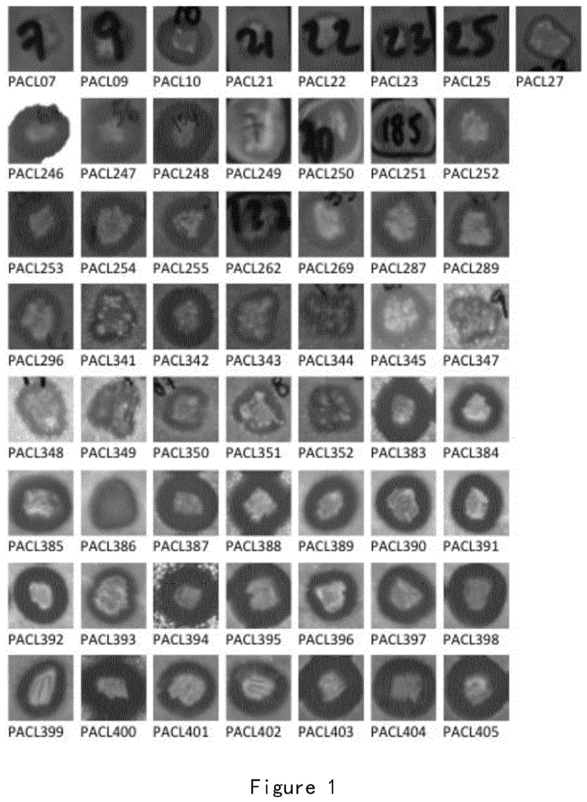

Propionibacterium acnes coverage test confirmed the antibacterial activity of lysin. Briefly, E. coli BL21 (DE3) clones containing the recombinant lysin plasmid were plated on LB agar containing IPTG for induction. Once the lysin is overexpressed, the E. coli clone osmotically releases the lysin. Soft agar with P. acnes embedded was overlaid and cultured to allow P. acnes to grow. The presence of active lysin forms a zone of clearing or halo around the E. coli. The experimental results are shown in Figure 1.

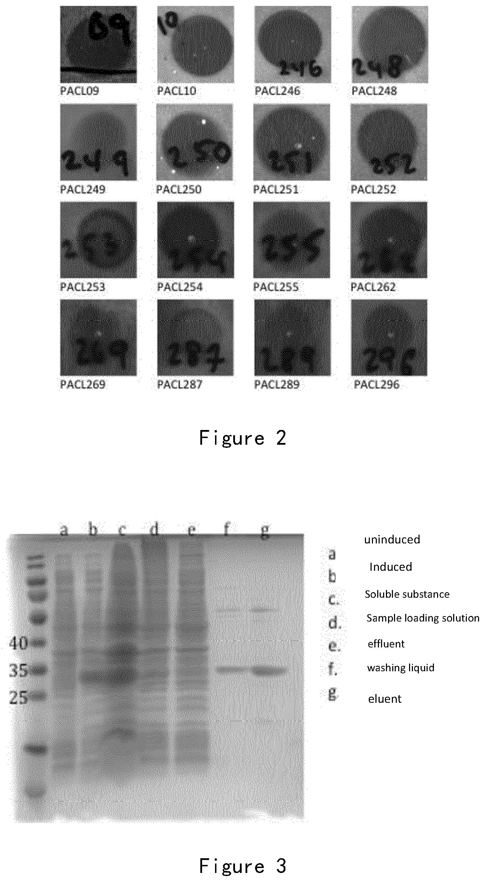

In a similar experiment, E. coli BL21 (DE3) clones containing the recombinant lysin plasmid were induced in liquid medium. Cells were harvested by centrifugation and sonicated in 50 mM sodium phosphate pH 7.4 buffer. The lysin is centrifuged to separate soluble and insoluble fractions. Soluble crude lysin were spotted on soft agar embedded with P. acnes. Active lysin in the soluble crude lysate formed a clearing zone after culturing to grow P. acnes. The experimental results are shown in Figure 2.

### Example 4

### Expression and purification of PACL10

The expression and purification of PACL10 were as follows. Briefly, Escherichia coli BL21 (DE3) containing the recombinant PACL10 expression plasmid was cultured in an autoinduction medium at 37°C and 300 rpm until the OD₆₀₀ reached 0.6-0.8, and then continued to culture at 18°C and 300 rpm for 16-18 hours. Cells were collected by centrifugation, resuspended in 50mM sodium phosphate pH 7.4, and homogeneously lysed under high pressure. The lysin was centrifuged again to collect the soluble crude lysin. The soluble fraction was mixed with an equal volume of 5 M NaCl, and the mixture was loaded onto a hydrophobic column. After sample loading, the column was washed with 5 column volumes of 20 mM sodium phosphate (pH 7.4), 2.5 M NaCl. PACL10 was then eluted with 10 mM sodium phosphate (pH 7.4). The experimental results are shown in Figure 3.

### Example 5

The chimeric lysin was expressed and purified in a similar manner to PACL10. Escherichia coli BL21 (DE3) containing recombinant chimeric lysin expression plasmid was cultured in self-inducing medium at 37°C and 300 rpm until the OD₆₀₀ reached 0.6-0.8, and then at 18°C and 300 rpm for continuous incubation for 16-18 hours. Cells are collected by centrifugation, resuspended in 50mM sodium phosphate pH 7.4, and lysed by homogenization under high pressure. The lysate was centrifuged again to collect the soluble crude lysate. The soluble fraction was mixed with an equal volume of 5 M NaCl, and the mixture was loaded onto a hydrophobic column. After sample loading, the column was washed with 5 column volumes of 20 mM sodium phosphate (pH 7.4), 2.5 M NaCl. The recombinant chimeric lysin was then eluted with 10 mM sodium phosphate (pH 7.4).

### Example 6

### Minimum Inhibitory Concentration Determination

The minimal inhibitory concentration (MIC) determination method is as follows. First prepare a colony suspension in 0.9% saline to an OD₆₀₀ of 0.05 to prepare an inoculum, which corresponds to 10⁷ colony-forming units per mL (cfu/mL). The suspension was diluted 20 times in agar-free enhanced Clostridium medium (RCM) to an inoculum of 5*10⁵cfu/mL. Prepare 2-fold serial dilutions of PACL10 or vancomycin with 0.9% saline and add no more than one-tenth of the total culture. Cultures were grown anaerobically at 37°C for 3 days. The MIC is the lowest concentration of PACL10 or vancomycin at which no growth of P. acnes can be observed with the naked eye. The minimum bactericidal concentration (MBC) was determined by subculturing the above cultures on enhanced Clostridium agar (RCA) plates containing 0.1% Tween-80. MBC is the lowest concentration of PACL10 or vancomycin at which no P. acnes colonies are seen on the enhanced Clostridium agar (RCA) plate.

Experimental results: Table 6-1 shows the minimal inhibitory concentration (MIC) and minimal bactericidal concentration (MBC) of PACL10 to 12 strains of Propionibacterium acnes bacterial strains from IA1, IA2, and II clades compared with vancomycin.

**Table 6-1**

| **Clade** | **Strai n** | **#10 (29380 g/mol)** | | | | **Vancomycin (1485.7 g/mol)** | | | |
|---|---|---|---|---|---|---|---|---|---|
| | | MIC | | MBC | | MIC | | MBC | |
| | | µg/mL | µM | µg/mL | µM | µg/mL | µM | µg/mL | µM |
| IA₁ | 1 | 6.4 | 0.218 | 25.6 | 0.871 | 0.5 | 0.337 | 0.5 | 0.337 |
| | 5 | 12.8 | 0.436 | 12.8 | 0.436 | 1.0 | 0.673 | 1.0 | 0.673 |
| | 7 | 6.4 | 0.218 | 12.8 | 0.436 | 0.5 | 0.337 | 1.0 | 0.673 |
| | 15 | 6.4 | 0.218 | 25.6 | 0.871 | 1.0 | 0.673 | 1.0 | 0.673 |
| | 27 | 12.8 | 0.436 | >25.6 | >0.871 | 1.0 | 0.673 | 1.0 | 0.673 |
| IA₂ | 10 | 6.4 | 0.218 | 12.8 | 0.436 | 0.5 | 0.337 | 1.0 | 0.673 |
| | 14 | 6.4 | 0.218 | >25.6 | >0.871 | 0.5 | 0.337 | 1.0 | 0.673 |
| | 17 | 6.4 | 0.218 | 25.6 | 0.871 | 0.5 | 0.337 | 0.5 | 0.337 |
| | 31 | 12.8 | 0.436 | >25.6 | >0.871 | 0.5 | 0.337 | 0.5 | 0.337 |
| | 33A | 12.8 | 0.436 | >25.6 | >0.871 | 0.5 | 0.337 | 1.0 | 0.673 |
| IB | 13 | 12.8 | 0.436 | 25.6 | 0.871 | 1.0 | 0.673 | 1.0 | 0.673 |
| II | 34A | 6.4 | 0.218 | 6.4 | 0.218 | 0.5 | 0.337 | 0.5 | 0.337 |

### Example 7

### Time sterilization test

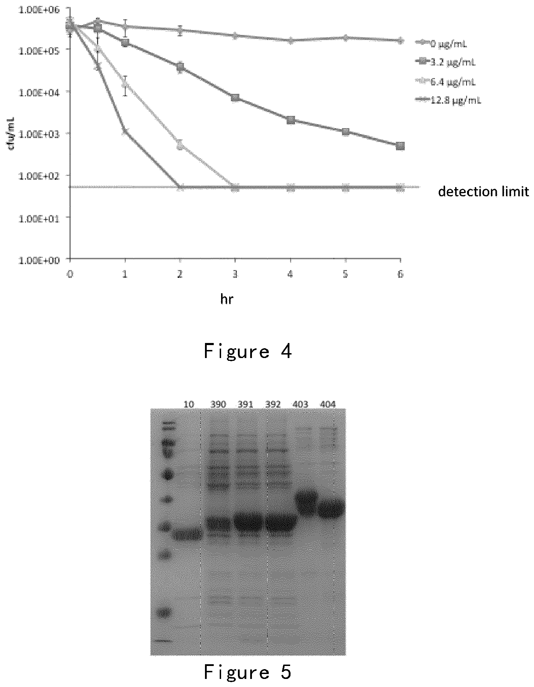

The time sterilization test method is as follows. Resuspend the P. acnes 34A strain colony in 0.9% saline to an OD₆₀₀ of 0.05, which corresponds to 10⁷ cfu/mL. The suspension was diluted 10-fold in 50 mM sodium phosphate (pH 6.0) to ~10⁶ cfu/mL. Add 0, 0.5x, 1x, 2x MIC of two-fold serial dilutions of PACL10 to the 34A strain, respectively. Ten-fold serial dilutions (0, 1, 2, 3 log) from the assay were plated on enhanced Clostridium agar (RCA) at each time point and incubated anaerobically at 37°C for 3 days. The experimental results are shown in Figure 4.

### Example 8

### Minimal inhibitory concentrations of selected lysin after single-step purification

Express PACL 10, 390, 391, 392, 403 and 404 according to the method of Examples 1-4, and perform the following single-step purification: put Escherichia coli BL21 (DE3) containing each recombinant expression plasmid in the self-induction medium incubate at 37°C, 300 rpm for 3 hours, then at 18°C, 300 rpm for 16-18 hours. Cells were collected by centrifugation and lysed by homogenizatoin under high pressure. Clarified lysate from 1 L of E. coli culture medium expressing the protein of interest was loaded onto a 26mm/200mm column containing 70 mL of purification resin. The lysin was purified according to the conditions in Table 8-1, and the purity determination of the lyase after single-step purification is shown in Figure 5. These lysin are used to determine the minimal inhibitory concentration (MIC).

The minimal inhibitory concentration (MIC) determination method is as follows. Propionibacterium acnes is streaked on Fortified Clostridium Agar (Fortified Clostridium Medium RCM with 1.5% agar, RCM was prepared according to the revised recipe, per liter - 10g acid hydrolyzed casein, 10g beef extract, 3g yeast extract, 5g D - glucose, 5 g sodium chloride, 3 g sodium acetate, 0.5 g L-cysteine hydrochloride). Plates were incubated anaerobically at 37°C for 72 hours. The inoculum for MIC determination was first prepared by resuspending single clones from RCA in 0.9% saline to an OD₆₀₀ of 0.05, corresponding to 10⁷ colony-forming units per milliliter (cfu/mL). The suspension was diluted 20-fold in RCM to an inoculum of 5*10⁵ cfu/mL. Two-fold serial dilutions of each lyase were prepared in 0.9% saline and added not to exceed one-tenth of the total culture volume. Cultures were grown anaerobically at 37°C for 48-72 hours. The MIC is the lowest concentration of lyase at which no growth of P. acnes can be observed with the naked eye. The MIC results are shown in Table 8-2.

**Table 8-1 Purification conditions of selected lysin**

| PACL | homogenization buffer | binding buffer | Chromatography | Elution buffer |
|---|---|---|---|---|
| 10 | sodium phosphate, pH 7.4 | 25 mM sodium phosphate, 2.5 M NaCl, pH 7.4 | Hydrophobic Interaction, Phenyl Sepharose (Low Substitution) | Gradient to 10 mM sodium phosphate |
| 390 | 50 mM sodium phosphate, pH 7.4 | 25 mM sodium phosphate, 2.5 M NaCl, pH 7.4 | Hydrophobic Interaction, Phenyl Sepharose (Low Substitution) | Gradient to 10 mM sodium phosphate |
| 391 | 50 mM sodium phosphate, pH 7.4 | 25 mM sodium phosphate, 2.5 M NaCl, pH 7.4 | Hydrophobic Interaction, Phenyl Sepharose (Low Substitution) | Gradient to 10 mM sodium phosphate |
| 392 | 50 mM sodium phosphate, pH 7.4 | 25 mM sodium phosphate, 2.5 M NaCl, pH 7.4 | Hydrophobic Interaction, Phenyl Sepharose (Low Substitution) | Gradient to 10 mM sodium phosphate |
| 403 | 20 mM sodium phosphate, pH 7.4 | 20 mM sodium phosphate, pH 7.4 | Ion exchange, SP Sepharose | Gradient to 20 mM NaP, 0.5 M NaCl, pH 7.4 |
| 404 | 20 mM sodium phosphate, pH 7.4 | 20 mM sodium phosphate, pH 7.4 | Ion exchange, SP Sepharose | Gradient to 20 mM NaP, 0.5 M NaCl, pH 7.4 |

**Table 8-2 MIC of selected lyases relative to Propionibacterium acnes (µg/mL)**

| Clade | Strain | PACL10 | PACL390 | PACL391 | PACL392 | PACL403 | PACL404 | Tetracycline |
|---|---|---|---|---|---|---|---|---|
| IA₁ | 1 | 8 | 8 | 8 | 8 | 16 | 8 | 8 |
| | 5 | 8 | 8 | 16 | 8 | 16 | 16 | 8 |
| | 7 | 8 | 8 | 8 | 8 | 16 | 16 | 8 |
| IA₂ | 10 | 8 | 8 | 32 | 16 | 8 | 16 | 8 |
| | 17 | 8 | 8 | 32 | 16 | 8 | 8 | 8 |
| | 33 | 8 | 8 | 16 | 8 | 8 | 8 | 8 |
| II | 34 | 8 | 8 | 8 | 8 | 16 | 8 | 8 |

### Example 9

### Purification and bactericidal activity of PACL392

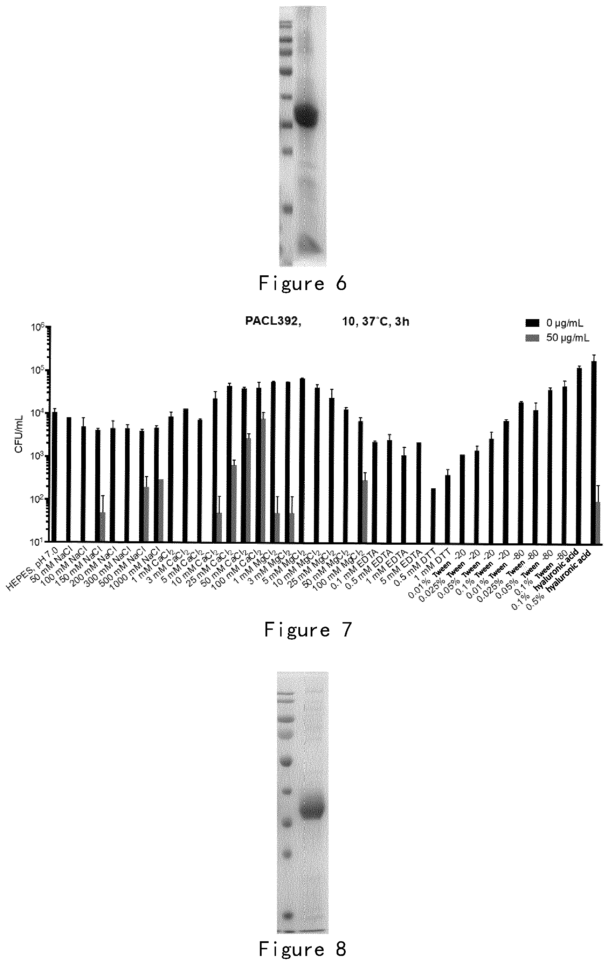

PACL392 was expressed in Escherichia coli BL21(DE3) containing the recombinant expression plasmid by placing it in autoinduction medium and culturing at 37°C, 300 rpm for 3h, followed by 18°C, 300 rpm for 16-18h. Cells were collected by centrifugation and homogeneously lysed in 20 mM sodium phosphate, pH 7.4, under high pressure. The lysate was centrifuged at 12,000 rpm, 4°C for 1 h. The supernatant was mixed with an equal volume of 20 mM sodium phosphate, pH 7.4, 1 M ammonium sulfate, and centrifuged again at 12,000 rpm, 4°C for 1 h. The supernatant was filtered with a 0.22 µm filter membrane and loaded onto a mixed-mode chromatographic column equilibrated with 20mM sodium phosphate, pH 7.4, and 0.5M ammonium sulfate (70 mL purified resin 26mm/200mm column). After sample loading, the column was washed with a series of buffers: 20 mM sodium phosphate, pH 7.4, 0.5 M ammonium sulfate; 20 mM sodium phosphate, pH 7.4, 2.5 M NaCl; 20 mM sodium phosphate, pH 7.4, and 50 mM, piperazine, pH 10.02, 50 mM NaCl. PACL392 was eluted with 50 mM piperazine, pH 10.02, and 750 mM NaCl. The eluate was dialyzed against 50 mM sodium phosphate, pH 7.4 to neutralize the pH and remove cations. Dialyzed samples were filtered and loaded onto an ion exchange column (70 mL of 26mm/200mm column of purification resin). The final result of PACL392 purification is shown in Figure 6.

The bactericidal activity of PACL392 was tested by 50 mM, pH 7.0 in the presence of a series of additives such as NaCl, CaCl₂, MgCl₂, EDTA, DTT, Tween-20, Tween-80 and hyaluronic acid (concentrations shown in Figure 7). CFU reduction in HEPES was determined.

### Example 10

### Purification and bactericidal activity of PACL403

PACL403 was expressed in Escherichia coli BL21(DE3) containing the recombinant expression plasmid by placing it in autoinduction medium and culturing at 37°C, 300 rpm for 3 h, followed by 18°C, 300 rpm for 16-18 h. Cells were collected by centrifugation and lysed by homogenization in 20 mM sodium phosphate, pH 7.4, under high pressure. The lysate solution was adjusted to 20 mM sodium phosphate, pH 7.4, 1 M NaCl, and centrifuged at 12,000 rpm, 4°C for 1 h. The supernatant was filtered with a 0.22 µm filter membrane and loaded onto a mixed-mode chromatographic column (26mm/200mm column of 70 mL purified resin) equilibrated with 20mM sodium phosphate, pH 7.4, and 1 M NaCl. After loading the column was washed with a series of buffers: 20 mM sodium phosphate, pH 7.4, 1 M NaCl; 20 mM sodium phosphate, pH 7.4, 2.5 M NaCl; 20 mM sodium phosphate, pH 7.4; 20 mM, Sodium phosphate pH 7.4, 0.1% Triton X-114 and again 20 mM sodium phosphate pH 7.4. PACL403 was eluted with 50 mM piperazine, pH 9.5, 1M NaCl. Neutralize the high pH by adding a fifth volume of 500 mM sodium phosphate, pH 7.4. The eluate was then dialyzed against 50 mM sodium phosphate, pH 7.4, to remove cations. Dialyze sample filtration. The final result of PACL403 purification is shown in Figure 8.

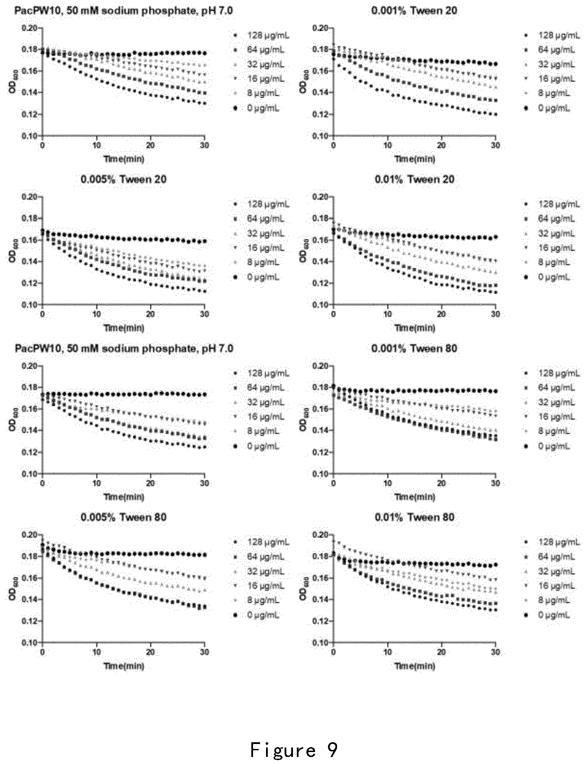

The bactericidal activity of PACL403 was determined by the reduction in OD in 50 mM sodium phosphate, pH 7.0, in the presence of Tween-20 and Tween-80 at the concentrations shown in FIG. 9.

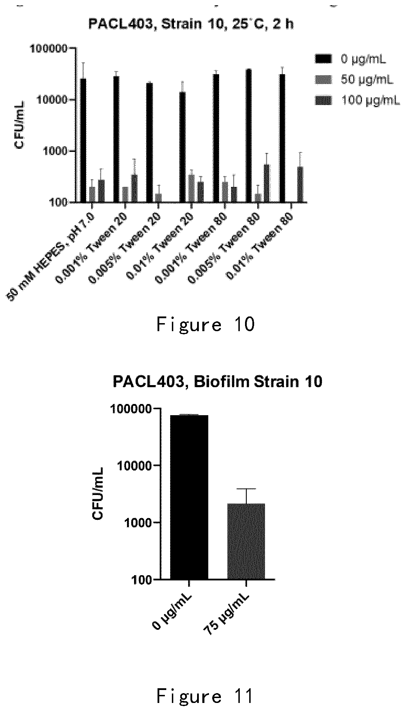

The bactericidal activity of PACL403 was determined by the reduction of CFU in 50 mM HEPES, pH 7.0, in the presence of Tween-20 and Tween-80 at the concentrations shown in FIG. 10.

PACL403 also killed biofilm-associated P. acnes (Fig. 11). Strain 10 with an OD₆₀₀ of 1.0 was added to a 96-well polystyrene plate to inoculate the biofilm overnight in an anaerobic gas mixture. The supernatant was removed and the wells were gently rinsed with 0.9% NaCl. Biofilm formation medium (RCM + 5% glucose) was added to allow biofilm growth overnight in the anaerobic gas mixture. The medium was removed and the wells were gently rinsed with 0.9% NaCl. Biofilms were then treated with buffer or PACL403 overnight at 25°C under anaerobic conditions. The buffer or PACL403 was removed and the wells were rinsed with 0.9% NaCl. Resuspend in 0.9% NaCl, plate on RCM agar and count biofilm-associated CFU.

## Claims

1. Use of a bacteriophage lysin or its chimera in the preparation of drugs, cosmetics, or medical devices for the prevention, treatment, or improvement of acne or infections caused by Propionibacterium acnes , or diseases related to Propionibacterium acnes, wherein the bacteriophage lysin comprises a bacteriophage lysin derived from the Nocardioidaceae or Propionibacteriaceae family.

2. The use according to claim 1, wherein the Nocardioidaceae bacteria include Micropruina, Propionicimonas, Propionicicella, Friedmanniella; the Propionibacteriaceae bacteria include Propioniferax, Mariniluteicoccus, Granulicoccus, Naumannella, Propioniciclava, Auraticoccus, Microlunatus, Aestuariimicrobium, Luteococcus, Tessaracoccus, Brooklawnia, Propionimicrobium, Propionibacterium, Cutibacterium, Acidipropionibacterium, or Pseudopropionibacterium;
Preferably Propionibacterium, Cutibacterium, Acidipropionibacterium, or Pseudopropionibacterium.

3. The use according to claim 1, wherein the bacteriophage lysin comprises lysins derived from Cutibacterium acnes, Propionibacterium humerusii, Cutibacterium avidum, Cutibacterium granulosum, Acidipropionibacterium thoenii, Acidipropionibacterium jensenii, Acidipropionibacterium acidipropionici, Aestuariimicrobium kwangyangense, Granulicoccus phenolivorans, Microlunatus phosphovorus, Pseudopropionibacterium propionicum, Tessaracoccus sp., Propionicicella superfundia, Propionibacterium freudenreichii, Propionibacterium freudenreichii subsp. Freudenreichii, Propionibacterium freudenreichii subsp. Shermanii, Propionibacterium acidifaciens, Propionibacterium lymphophilum, Propionibacteriaceae bacterium, Propionibacterium sp. oral taxon 192, Propioniferax innocua, Naumannella halotolerans, Propioniciclava tarda, Micropruina glycogenica, Propionicimonas paludicola, Auraticoccus monumenti, Luteococcus japonicus, Tessaracoccus oleiagri, Tessaracoccus bendigoensis, Tessaracoccus lapidicaptus, Acidipropionibacterium microaerophilum, Acidipropionibacterium olivae, or Acidipropionibacterium damnosum.

4. The use according to claim 1, wherein the bacteriophage lysin comprises lysin derived from Acidipropionibacterium jensenii, Acidipropionibacterium thoenii, Acidipropionibacterium acidipropionici, Acidipropionibacterium microaerophilum, Acidipropionibacterium olivae, Acidipropionibacterium damnosum, Cutibacterium acnes, Cutibacterium avidum, Cutibacterium granulosum, or Pseudopropionibacterium propionicum..

5. The use according to claim 1, wherein the bacteriophage lysin has the amino acid sequence shown in any one of SEQ ID NO: 1 to SEQ ID NO: 28.

6. The use according to claim 1, wherein the bacteriophage lysin has the amino acid sequence shown in SEQ ID NO:10.

7. The use according to claim 1 wherein the bacteriophage lysin has the nucleotide sequence shown in any one of SEQ ID NO:29 to SEQ ID NO:56.

8. The use according to claim 1, wherein the bacteriophage lysin has the nucleotide sequence shown in SEQ ID NO:38.

9. The use according to claim 1, wherein the chimera comprises the catalytic domain of bacteriophage lysin, or a combination of the catalytic domain and the binding domain of bacteriophage lysin.

10. The use according to claim 9, wherein the catalytic domain has a full C-terminal linker, a half C-terminal linker, no C-terminal linker, or any part of the linker; the binding domain has a full N-terminal linker, a half N-terminal linker, no N-terminal linker, or any portion of the linker.

11. The use according to claim 9, wherein the catalytic domain comprises one, two or more than two catalytic domains.

12. The use according to claim 9, wherein the binding domain comprises one, two or more than two binding domains.

13. The use according to claim 9, wherein the chimera further comprises a linker between the catalytic domain and the binding domain, the linker comprising:
1 ) The amino acid sequence shown in any one of SEQ ID NO:343~SEQ ID NO: 367;
2) The amino acid sequence shown in any one of SEQ ID NO:368~SEQ ID NO: 392;
3 ) the amino acid sequence shown in any one of SEQ ID NO:393~SEQ ID NO: 406; or
4 ) (GGGS)n, (GGGGS)n, (GGGGGS)n, (Gly)3-8, (EAAAK)n, (Ala-Pro)n, or A(EAAAK)nALEA(EAAAK)nA, where 1≤n≤15, n is an integer.

14. The use according to claim 9, wherein said chimera comprises said catalytic domain having the amino acid sequence shown in SEQ ID NO: 57, SEQ ID NO: 58, SEQ ID NO: 59, SEQ ID NO: 61, SEQ ID NO: 61, SEQ ID NO: ID NO:62, SEQ ID NO:63, SEQ ID NO:64, SEQ ID NO:65, SEQ ID NO:66, SEQ ID NO:67, SEQ ID NO:69, SEQ ID NO:70, SEQ ID NO :71, SEQ ID NO:73, SEQ ID NO:74, SEQ ID NO:77, SEQ ID NO:80, SEQ ID NO:81, SEQ ID NO:85, SEQ ID NO:86, SEQ ID NO:87 , SEQ ID NO: 91, SEQ ID NO: 93, SEQ ID NO: 95, SEQ ID NO: 97, SEQ ID NO: 98, SEQ ID NO: 99, SEQ ID NO: 100, SEQ ID NO: 102, SEQ ID NO: 102, SEQ ID NO: ID NO: 104, SEQ ID NO: 105, SEQ ID NO: 106, SEQ ID NO: 107, SEQ ID NO: 108, SEQ ID NO: 110, SEQ ID NO: 111, SEQ ID NO: 113, SEQ ID NO : 114, SEQ ID NO: 117, SEQ ID NO: 118, SEQ ID NO: 121, SEQ ID NO: 122, SEQ ID NO: 125, SEQ ID NO: 127, SEQ ID NO: 128, SEQ ID NO: 131 , SEQ ID NO:132, SEQ ID NO:133, SEQ ID NO:135, or SEQ ID NO:136;
The binding domain has the amino acid sequence shown in SEQ ID NO:60, SEQ ID NO:62, SEQ ID NO:68, SEQ ID NO:72, SEQ ID NO:75, SEQ ID NO:76, SEQ ID NO:78, SEQ ID NO:79, SEQ ID NO:82, SEQ ID NO:83, SEQ ID NO:84, SEQ ID NO:88, SEQ ID NO:89, SEQ ID NO:90, SEQ ID NO:92, SEQ ID NO: 94. SEQ ID NO: 96, SEQ ID NO: 101, SEQ ID NO: 103, SEQ ID NO: 109, SEQ ID NO: 112, SEQ ID NO: 115, SEQ ID NO: 116, SEQ ID NO: 119, SEQ ID NO: 120, SEQ ID NO: 123, SEQ ID NO: 124, SEQ ID NO: 126, SEQ ID NO: 129, SEQ ID NO: 130, SEQ ID NO: 134, SEQ ID NO: 137, SEQ ID NO:138 or SEQ ID NO:139.

15. The use according to claim 9, wherein the catalytic domain has the nucleotide sequence shown in SEQ ID NO: 140, SEQ ID NO: 141, SEQ ID NO: 142, SEQ ID NO: 144, SEQ ID NO: 146, SEQ ID NO: 146, SEQ ID NO: ID NO: 147, SEQ ID NO: 148, SEQ ID NO: 149, SEQ ID NO: 150, SEQ ID NO: 152, SEQ ID NO: 153, SEQ ID NO: 154, SEQ ID NO: 156, SEQ ID NO : 157, SEQ ID NO: 160, SEQ ID NO: 163, SEQ ID NO: 164, SEQ ID NO: 168, SEQ ID NO: 169, SEQ ID NO: 170, SEQ ID NO: 174, SEQ ID NO: 176 , SEQ ID NO: 178, SEQ ID NO: 180, SEQ ID NO: 181, SEQ ID NO: 182, SEQ ID NO: 183, SEQ ID NO: 185, SEQ ID NO: 187, SEQ ID NO: 188, SEQ ID NO: 188, SEQ ID NO: ID NO: 189, SEQ ID NO: 190, SEQ ID NO: 191, SEQ ID NO: 193, SEQ ID NO: 194, SEQ ID NO: 196, SEQ ID NO: 197, SEQ ID NO: 200, SEQ ID NO :201, SEQ ID NO:204, SEQ ID NO:205, SEQ ID NO:208, SEQ ID NO:210, SEQ ID NO:211, SEQ ID NO:214, SEQ ID NO:215, SEQ ID NO:216 , SEQ ID NO:218, or SEQ ID NO:219;
The binding domain has the nucleotide sequence shown in SEQ ID NO: 143, SEQ ID NO: 145, SEQ ID NO: 151, SEQ ID NO: 155, SEQ ID NO: 158, SEQ ID NO: 159, SEQ ID NO: 161, SEQ ID NO: 162, SEQ ID NO: 165, SEQ ID NO: 166, SEQ ID NO: 167, SEQ ID NO: 171, SEQ ID NO: 172, SEQ ID NO: 173, SEQ ID NO: 175, SEQ ID NO: 177. SEQ ID NO: 179, SEQ ID NO: 184, SEQ ID NO: 186, SEQ ID NO: 192, SEQ ID NO: 195, SEQ ID NO: 198, SEQ ID NO: 199, SEQ ID NO: 202, SEQ ID NO:203, SEQ ID NO:206, SEQ ID NO:207, SEQ ID NO:209, SEQ ID NO:212, SEQ ID NO:213, SEQ ID NO:217, SEQ ID NO:220, SEQ ID NO:221, or SEQ ID NO:222.

16. The use according to claim 9, wherein the described chimera has the amino acid sequence shown in any one of SEQ ID NO:223 ~ SEQ ID NO:282; Preferably the amino acid sequence shown in SEQ ID NO:258, SEQ ID in NO:259, SEQ ID NO:260, SEQ ID NO:271 or SEQ ID NO:272; more preferably the amino acid sequence shown in SEQ ID NO:260 or SEQ ID NO:271.

17. The use according to claim 9, wherein the chimera has a nucleotide sequence shown in any one of SEQ ID NO:283 ~ SEQ ID NO:342; preferably the nucleotide sequence shown in SEQ ID NO:316, SEQ ID NO:317, SEQ ID NO:318, SEQ ID NO:329 or SEQ ID NO:330; more preferably the nucleotide sequence shown in SEQ ID NO:318 or SEQ ID NO:329.

18. The use according to claim 1, wherein the infection caused by Propionibacterium acnes includes invasive infection, postoperative infection and/or instrument-related infection.

19. The use according to claim 18, wherein the device-related infection includes joint prosthesis, shunt tube and artificial heart valve-related infection.

20. The use according to claim 18, wherein the infection includes bone and/or joint infection, especially postoperative shoulder infection, as well as oral cavity, eye, intervertebral disc and brain infection.

21. The use according to claim 1, wherein the diseases related to Propionibacterium acnes include prostatitis leading to cancer, SAPHO (synovitis, acne, impetigo, hypertrophy, osteitis) syndrome syndrome, sarcoidosis, or sciatica.

22. The use according to claim 1, wherein the medical device comprises any device for releasing the lysin or chimera thereof to the affected area, preferably a clamp or patch applied to the skin surface or sprays, devices that use microneedles to enhance skin penetration of lysin or their chimeras, fine needles used by cosmetic professionals to apply lysin or their chimeras specifically to acne-affected hair follicles, or other similar device.

23. The use according to claim 1, wherein the medical device comprises a prosthetic device that will immobilize the lysin or its chimera in a location susceptible to P. acnes infection, preferably for a particularly susceptible infection Prosthetic implants in shoulder surgery for Propionibacterium acnes.

24. A bacteriophage lysin chimera in the application of any one of claims 1 to 23, said chimera has an amino acid sequence shown in any one of SEQ ID NO:223~SEQ ID NO:282; preferably the amino acid sequence shown in SEQ ID NO:258, SEQ ID NO:259, SEQ ID NO:260, SEQ ID NO:271 or SEQ ID NO:272; more preferably the amino acid sequence shown in SEQ ID NO:260 or SEQ ID NO:271.

25. The chimera according to claim 24, wherein the chimera has a nucleotide sequence shown in any one of SEQ ID NO:283 ~ SEQ ID NO:342; preferably the nucleotide sequence shown in SEQ ID NO:316, SEQ ID NO:317, SEQ ID NO:318, SEQ ID NO:329 or SEQ ID NO:330; more preferably the nucleotide sequence shown in SEQ ID NO:318 or SEQ ID NO:329.

26. A method for preparing the chimera described in any one of claims 1 to 23 or any one of claims 24 to 25, wherein the method comprises:
(1) Synthetic domain sequences and primers for amplifying domain sequences;
(2) Using Taq DNA polymerase PCR to amplify the domain sequence;
(3) The PCR product was gel purified and ligated with the expression plasmid pET28
(4) Transfer the recombinant plasmid to Escherichia coli BL21 (DE3);
(5) Cultivate Escherichia coli BL21 (DE3) containing the recombinant plasmid, induce expression, collect the cells by centrifugation, lyse, and purify to obtain the chimera.

27. The method according to claim 26, further comprises:
Escherichia coli BL21 (DE3) containing recombinant chimeric lysin expression plasmid was cultured in self-inducing medium at 37°C and 300 rpm until the OD₆₀₀ reached 0.6-0.8, and then at 18°C and 300 rpm for continuous cultivate for 16-18 hours; collect the cells by centrifugation, resuspend in 50mM sodium phosphate pH 7.4, and homogeneously lyse under high pressure; centrifuge the lysate again to collect the soluble crude lysate. The soluble fraction was mixed with an equal volume of 5 M NaCl, and the mixture was loaded onto a hydrophobic column; after loading, the column was washed with 5 times the column volume of 20 mM sodium phosphate (pH 7.4), 2.5 M NaCl; then washed with 10 mM Sodium phosphate (pH 7.4) eluted the recombinant chimeric lysin.

28. preparations containing the bacteriosphage lysin or its chimera in application according to any one of claims 1 to 23, or a chimera according to any one of claims 24 to 25, wherein the preparations further comprise antibiotics, other lysin, or inactive excipients.

29. The amino acid sequence encoding the phage lysin or its chimera used in any one of claims 1 to 23, or the chimera described in any one of claims 24 to 25, or the chimera prepared in claim 26, or an amino acid sequence with a similarity of 80% and above, 85% and above, 90% and above, 95% and above, or 99% and above, or an alternative amino acid sequence with the same functional group.

30. The amino acid sequence according to claim 29, wherein the replacement amino acid sequence is a conservative substitution using amino acids in the same group of amino acids, the amino acid group comprising:
Aliphatic: glycine, alanine, valine, leucine or isoleucine;
Hydroxyl or sulfur/selenium containing: serine, cysteine, threonine or methionine;
Cyclic: proline;
Aromatic: phenylalanine, tyrosine or tryptophan;
Basic: histidine, lysine, or arginine; or
Acidic and their amides: aspartic acid, glutamic acid, asparagine, glutamine.

31. Nucleotide sequence encoding the phage lysin or its chimera in any one of claims 1 to 23, or the chimera of any one of claims 24 to 25, or the chimera prepared in claim 26, or its synonymous codon sequence.

32. The bacteriophage lysin or its chimera in the application of any one of claims 1 to 23, or the chimera described in any one of claims 24 to 25, or the chimera prepared by claim 26 is used for bacterial lysis application of reagents, wherein said bacterial lysates are used for DNA extracting and typing using a PCR-based kit.

33. The bacteriophage lysin or its chimera in the application of any one of claims 1 to 23, or the chimera of any one of claims 24 to 25, or the chimera prepared by claim 26 as a application of a disinfectant or sterilant on abiotic surfaces, wherein said disinfectant or sterilant prevents infection by removing P. acnes in planktonic form or in biofilms, preferably on surgical equipment during surgery or prosthetic implants.

34. Use of The phage lysin or its chimera in any one of claims 1 to 23, or the chimera of any one of claims 24 to 25, or the chimera prepared in claim 26, or a single binding domain in the above-mentioned molecule, or a series combination of similar or different binding domains in the above-mentioned molecules, in the preparation of a diagnostic tool for Propionibacterium acnes, wherein said the lysin, Chimera or binding domains are used in combination with signaling molecules.

35. The use according to claim 34, wherein the fusion of the lysin, chimera or binding domain and the signal molecule is formed by gene fusion or chemical coupling.

36. The use according to claim 35, wherein the fusion is used to directly detect Propionibacterium acnes on a microscope slide by fluorescence or other means, for marking Propionibacterium acnes by immunohistochemistry, and use as a detection reagent in ELISA assaysand on Western blot, for attachment to magnetic beads in MACS or other pull-down assays, or as a detection reagent in assays where antibodies are used as detection reagents.

37. The use according to claim 34, wherein the signal molecules include proteins or chemical fluorescent dyes, protein tags, enzymes, avidin, streptavidin, ovalbumin, biotin, para Click chemical label-sensitive tags, inteins, or other molecules that can cause the recruitment of secondary proteins or molecules that produce signals,
The fluorescent dyes include GFP, RFP, mCherry, FITC, TRITC, Alexafluor 488, Cy3 or Cy5;
The protein tag includes Flag-tag, myc-tag, halo-tag, his-tag, or any other tag that can be combined with antibodies or other high-affinity molecules to generate signals;
The enzymes include firefly luciferase, beta-lactamase, alkaline phosphatase, horseradish peroxidase, or any reaction that causes a reaction such as light, color change, substrate deposition, or other enzymes that can be detected in the assay.

38. The Use of bacteriophage lysin or its chimera of any one of claims 1 to 23, or the chimera of any one of claims 24 to 25, or the catalitic domain in the chimera prepared by method of claim 26in the preparation of medicines for the treatment of P. acnes infection, wherein the catalytic domain is combined with a targeting module.
